# EUROPÄISCHE PATENTSCHRIFT

(11) **EP 1 654 211 B1**
(45) Veröffentlichungstag und Bekanntmachung des Hinweises auf die Patenterteilung: **15.05.2013**
(21) Anmeldenummer: 04763465.4
(22) Anmeldetag: 23.07.2004
(51) Int. Cl.: C07C 51/265, C07C 51/31, B01J 23/22, B01J 23/50

(54) **SILBER, VANADIUM UND EIN PROMOTORMETALL ENTHALTENDES MULTIMETALLOXID UND DESSEN VERWENDUNG**
MULTI-METAL OXIDE CONTAINING SILVER, VANADIUM AND A PROMOTER METAL AND USE THEREOF
OXYDE POLYMETALLIQUE CONTENANT DE L'ARGENT, DU VANADIUM ET UN METAL PROMOTEUR ET SON UTILISATION

(30) Priorität: 25.07.2003 DE 10334132
(43) Veröffentlichungstag der Anmeldung: 10.05.2006
(73) Patentinhaber: BASF SE, 67056 Ludwigshafen (DE)
(72) Erfinder: NETO, Samuel, 68161 Mannheim (DE); HIBST, Hartmut, 69198 Schriesheim (DE); ROSOWSKI, Frank, 68165 Mannheim (DE); STORCK, Sebastian, 68167 Mannheim (DE)
(86) Internationale Anmeldenummer: PCT/EP2004/008296
(87) Internationale Veröffentlichungsnummer: WO 2005/012216

(56) Entgegenhaltungen:
- EP-A- 0 447 267
- EP-A- 0 522 871
- WO-A-00/27753
- WO-A-01/85337
- DE-A- 19 705 326
- US-A- 4 137 259
- HOLLES J H ET AL: "A substrate-versatile catalyst for the selective oxidation of light alkanes - I. Reactivity" JOURNAL OF CATALYSIS, ACADEMIC PRESS, DULUTH, MN, US, Bd. 218, Nr. 1, 19. März 2003 (2003-03-19), Seiten 42-53, XP004432693 ISSN: 0021-9517

## Beschreibung

Die Erfindung betrifft ein Silber, Vanadium und ein Promotormetall enthaltendes Multimetalloxid, dessen Verwendung zur Herstellung von Präkatalysatoren und Katalysatoren zur Gasphasenpartialoxidation von aromatischen Kohlenwasserstoffen, die so erhaltenen (Prä)katalysatoren und ein Verfahren zur Herstellung von Aldehyden, Carbonsäuren und/oder Carbonsäureanhydriden unter Verwendung der Katalysatoren.

Bekanntermaßen wird eine Vielzahl von Aldehyden, Carbonsäuren und/oder Carbonsäureanhydriden technisch durch die katalytische Gasphasenoxidation von aromatischen Kohlenwasserstoffen wie Benzol, o-, m- oder p-Xylol, Naphthalin, Toluol oder Durol (1,2,4,5-Tetramethylbenzol) in Festbettreaktoren, vorzugsweise Rohrbündelreaktoren, hergestellt. Dabei werden je nach Ausgangsmaterial beispielsweise Benzaldehyd, Benzoesäure, Maleinsäureanhydrid, Phthalsäureanhydrid, Isophthalsäure, Terephthalsäure oder Pyromellithsäureanhydrid gewonnen. Dazu wird im Allgemeinen ein Gemisch aus einem molekularen Sauerstoff enthaltenden Gas, beispielsweise Luft und das zu oxidierende Ausgangsmaterial durch eine Vielzahl in einem Reaktor angeordneter Rohre geleitet, in denen sich eine Schüttung mindestens eines Katalysators befindet.

In der WO 00/27753 und der WO 01/85337 werden Silber- und Vanadiumoxid enthaltende Multimetalloxide und deren Verwendung für die partielle Oxidation von aromatischen Kohlenwasserstoffen beschrieben.

Die DE 197 05 326 offenbart Verfahren zur selektiven Oxidation von nicht-cyclischen Kohlenwasserstoffen mittels Katalysatoren auf Basis von Silber-Vanadium-Mischoxiden. Ein Schalenkatalysator mit 5,2 Gew.-% V₂O₅, 0,5 Gew.-% Ag und 0,02 Gew.% Cs und analog hergestellte Katalysatoren der Struktur AgₓM_{y}VO_{z} sind beschrieben.

Die US 4,137,259 beschreibt eine Katalysatormasse aus Silbervanadat und Eisenvanadat, die zur Gasphasenoxidation von Toluol zu Benzaldehyd und/oder Benzoesäure verwendet wird. Ein Vergleichsbeispiel betrifft eine Masse aus Silbervanadat und Cervanadat mit einem Atomverhältnis Ag:Ce:V von 2:1:5.

Holles J. H. et al., Journal of Catalysis 218 (2003) 42-53 erwähnen ein gemischtes Metalloxid der Formel Ag_{1,2}V₃Ce_{0,15}V₈₊ₓ.

Der Erfindung liegt die Aufgabe zu Grunde, die mit diesen Katalysatoren erreichten Ausbeuten ohne Beeinträchtigung der Selektivitäten zu verbessern.

Die Aufgabe wird erfindungsgemäß durch Multimetalloxide der allgemeinen Formel I gelöst,

Ag_{a-c}Q_{b}M_{c}V₂O_{d}*eH₂O, I

worin
- a: einen Wert von 0,6 bis 0,9 hat,
- Q: für ein unter P, As, Sb und/oder Bi ausgewähltes Element steht,
- b: einen Wert von 0 bis 0,3 hat,
- M: für ein unter Ce und/oder Mn ausgewähltes Metall steht,
- c: einen Wert von 0,01 bis 0,1 hat,
- d: eine Zahl, die sich durch die Wertigkeit und Häufigkeit der von Sauerstoff verschiedenen Elemente in der Formel I bestimmt, bedeutet und
- e: einen Wert von 0 bis 20 hat.

Das erfindungsgemäße Multimetalloxid liegt in einer Kristallstruktur vor, deren Pulverröntgendiagramm durch Beugungsreflexe bei den Netzebenenabständen d 15,23 ± 0,6, 12,16 ±0,4, 10,68 ± 0,3, 3,41 ± 0,04, 3,09 ± 0,04, 3,02 ± 0,04, 2,36 ± 0,04 und 1,80 ±0,04 Å gekennzeichnet ist.

Die Erfindung betrifft außerdem einen Präkatalysator, der in einen Katalysator zur Gasphasenpartialoxidation von aromatischen Kohlenwasserstoffen umwandelbar ist und aus einem inerten nicht-porösen Träger und wenigstens einer darauf aufgebrachten Schicht besteht, die ein oben definiertes Multimetalloxid umfasst.

Die Erfindung betrifft außerdem einen Katalysator zur Gasphasenpartialoxidation von aromatischen Kohlenwasserstoffen, der aus einem inerten nicht-porösen Träger und wenigstens einer darauf aufgebrachten Schicht besteht, die als katalytisch aktive Masse eine Silber-Vanadiumoxid-Bronze umfasst, die wenigstens ein unter Ce und/oder Mn ausgewähltes Metall M enthält, und aus einer oben definierten Multimetalloxidmasse oder einem oben definierten Präkatalysator durch thermische Behandlung bei Temperaturen oberhalb 200 °C bis 650 °C herstellbar ist.

Die Erfindung betrifft außerdem ein Verfahren zur Herstellung von Aldehyden, Carbonsäuren und/oder Carbonsäureanhydriden, bei dem man einen gasförmigen Strom, der einen aromatischen Kohlenwasserstoff und ein molekularen Sauerstoff enthaltendes Gas umfasst, bei erhöhter Temperatur mit einem vorstehend definierten Katalysator in Kontakt bringt.

Die Angabe der Röntgenbeugungsreflexe erfolgt in dieser Anmeldung in Form der von der Wellenlänge der verwendeten Röntgenstrahlung unabhängigen Netzebenenabstände d[Å], die sich aus dem gemessenen Beugungswinkel mittels der Bragg'schen Gleichung errechnen lassen.

Im Multimetalloxid der Formel I hat die Variable a einen Wert von 0,6 bis 0,9, der Wert der Variablen b beträgt vorzugsweise 0 bis 0,1, und der Wert der Variablen c beträgt 0,01 bis 0,1.

Die Zahl d bestimmt sich aus der Wertigkeit und Häufigkeit der von Sauerstoff verschiedenen Elemente im Multimetalloxid der Formel I. Die Zahl e, die ein Maß für den Wassergehalt ist, beträgt vorzugsweise 0 bis 5.

Die spezifische Oberfläche nach BET, gemessen gemäß DIN 66 131, die auf den "Recommendations 1984" der IUPAC International Union of Pure and Applied Chemistry (s. Pure & Appl. Chem. 57, 603 (1985)) basiert, beträgt in der Regel mehr als 1 m²/g, bevorzugt 3 bis 250 m²/g, insbesondere 10 bis 250 m²/g und besonders bevorzugt 20 bis 80 m²/g_{.}

Als Metalle M sind Nb, Ce, W, Mn und Ta, insbesondere Ce und Mn, bevorzugt, wovon Ce am meisten bevorzugt ist.

Zur Herstellung der erfindungsgemäßen Multimetalloxide wird im Allgemeinen eine Suspension von Vanadiumpentoxid (V₂O₅) mit der Lösung einer Silberverbindung und einer Lösung einer Verbindung der Metallkomponente M sowie gegebenenfalls der Lösung einer Verbindung von Q erhitzt. Als Lösungsmittel für diese Umsetzung können polare organische Lösungsmittel, wie Polyole, Polyether oder Amine, z. B. Pyridin, dienen, bevorzugt wird als Lösungsmittel Wasser verwendet. Als Silbersalz wird bevorzugt Silbernitrat verwendet, die Verwendung anderer löslicher Silbersalze, z. B. Silberacetat, Silberperchlorat oder Silberfluorid ist ebenfalls möglich.

Sofern mitverwendet, können das oder die Elemente Q aus der Gruppe P, As, Sb und/oder Bi in elementarer Form oder als Oxide oder Hydroxide eingesetzt werden. Vorzugsweise werden sie jedoch in Form ihrer löslichen Verbindungen, vor allem ihrer organischen oder anorganischen wasserlöslichen Verbindungen eingesetzt. Besonders bevorzugt sind hierunter die anorganischen wasserlöslichen Verbindungen, vor allem die Alkali- und Ammoniumsalze und insbesondere die teilneutralisierten oder freien Säuren dieser Elemente wie Phosphorsäure, Arsenwasserstoffsäure, Antimonwasserstoffsäure, die Ammoniumhydrogenphosphate, -arsenate, -antimonate und -bismutate und die Alkalihydrogenphosphate, -arsenate, -antimonate und -bismutate. Ganz besonders bevorzugt verwendet man als Element Q Phosphor für sich allein, insbesondere in Form von Phosphorsäure, phosphoriger Säure, hypophosphoriger Säure, Ammoniumphosphat oder Phosphorsäureester und vor allem als Ammoniumdihydrogenphosphat.

Als Salze der Metallkomponente M werden in der Regel solche gewählt, die im verwendeten Lösungsmittel löslich sind. Wird Wasser als Lösungsmittel bei der Herstellung der erfindungsgemäßen Multimetalloxide verwendet, können beispielsweise die Perchlorate oder Carboxylate, insbesondere die Acetate, der Metallkomponente M eingesetzt werden. Bevorzugt werden die Nitrate der betreffenden Metallkomponente M verwendet, insbesondere Cernitrat oder Mangannitrat.

Die Umsetzung des V₂O₅ mit der Silberverbindung, der Verbindung der Metallkomponente M und gegebenenfalls Q kann im Allgemeinen bei Raumtemperatur oder bei erhöhter Temperatur durchgeführt werden. In der Regel wird die Umsetzung bei Temperaturen von 20 bis 375 °C, vorzugsweise bei 20 bis 100 °C und besonders bevorzugt bei 60 bis 100 °C vorgenommen. Liegt die Temperatur der Umsetzung oberhalb der Temperatur des Siedepunktes des verwendeten Lösungsmittels, wird die Umsetzung zweckmäßigerweise unter dem Eigendruck des Reaktionssystems in einem Druckgefäß ausgeführt. Vorzugsweise werden die Reaktionsbedingungen so gewählt, dass die Umsetzung bei Atmosphärendruck durchgeführt werden kann. Die Dauer dieser Umsetzung kann in Abhängigkeit von der Art der umgesetzten Ausgangsmaterialien und den angewandten Temperaturbedingungen 10 Minuten bis 3 Tage betragen. Eine Verlängerung der Reaktionszeit der Umsetzung, beispielsweise auf 5 Tage und mehr, ist möglich. In der Regel wird die Umsetzung des V₂O₅ mit der Silberverbindung, der Verbindung der Metallkomponente M zum erfindungsgemäßen Multimetalloxid während eines Zeitraums von 6 bis 24 Stunden durchgeführt. Bei der Umsetzung verändert sich die orangerote Farbe der V₂O₅-Suspension und es bildet sich die neue Verbindung in Form einer dunkelbraunen Suspension.

Je nach der gewünschten chemischen Zusammensetzung des Multimetalloxids der Formel I werden zu dessen Herstellung die sich aus a und c von Formel I ergebenden Mengen von V₂O₅, Silberverbindung sowie der Verbindung der Metallkomponente M miteinander umgesetzt. So wird im Allgemeinen die Silberverbindung mit dem Vanadiumpentoxid in einem Mengenverhältnis umgesetzt, das einem Atomverhältnis Ag : V von 0,15 bis 0,95, vorzugsweise von 0,25 bis 0,5 entspricht, entsprechend einem Wert für a in Formel I von 0,3 bis 1,9 bzw. 0,5 bis 1,0. Besonders bevorzugt wird die Silberverbindung bezüglich des Vanadiumpentoxids in einer Menge zugesetzt, die einem Atomverhältnis Ag : V von 0,3 bis 0,45 entspricht, entsprechend einem Wert für a in Formel I von 0,6 bis 0,9. Die Verbindung der Metallkomponente M wird im Allgemeinen in einer Menge von 0,0005 bis 0,25, vorzugsweise 0,001 bis 0,1, bezogen auf V₂O₅, eingesetzt. Nach beendeter Umsetzung wird dabei das erfindungsgemäße Multimetalloxid mit faserförmiger Kristallmorphologie erhalten.

Das so gebildete erfindungsgemäße Multimetalloxid kann aus der Reaktionsmischung isoliert und bis zur weiteren Verwendung gelagert werden. Die Isolierung des Multimetalloxids kann z. B. durch Abfiltrieren der Suspension und Trocknen des erhaltenen Feststoffs erfolgen, wobei die Trocknung sowohl in herkömmlichen Trocknem, aber auch z. B. in Gefriertrocknern durchgeführt werden kann. Besonders vorteilhaft wird die Trocknung der erhaltenen Multimetalloxid-Suspension mittels Sprühtrocknung durchgeführt. Es kann vorteilhaft sein, das bei der Umsetzung erhaltene Multimetalloxid vor dessen Trocknung salzfrei zu waschen. Die Sprühtrocknung wird im Allgemeinen unter Atmosphärendruck oder vermindertem Druck vorgenommen. Je nach angewandtem Druck und verwendetem Lösungsmittel bestimmt sich die Eingangstemperatur des Trocknungsgases - im Allgemeinen wird als solches Luft verwendet, es können aber selbstverständlich auch andere Trocknungsgase wie Stickstoff oder Argon, benutzt werden. Die Eingangstemperatur des Trocknungsgases in den Sprühtrockner wird vorteilhaft so gewählt, dass die Ausgangstemperatur des durch Verdampfung des Lösungsmittels abgekühlten Trocknungsgases 200 °C für einen längeren Zeitraum nicht übersteigt. In der Regel wird die Ausgangstemperatur des Trocknungsgases auf 50 bis 150°C, vorzugsweise 100 bis 140 °C eingestellt. Falls eine Lagerung des Multimetalloxids nicht beabsichtigt ist, kann die erhaltene Multimetalloxid-Suspension auch ohne vorherige Isolierung und Trocknung des Multimetalloxids der weiteren Verwendung zugeführt werden, beispielsweise zur Herstellung der erfindungsgemäßen Präkatalysatoren durch Beschichtung.

Die erfindungsgemäßen Multimetalloxide werden als Vorläuferverbindung zur Herstellung der katalytisch aktiven Masse von Katalysatoren, wie sie zur Gasphasenoxidation von aromatischen Kohlenwasserstoffen zu Aldehyden, Carbonsäuren und/oder Carbonsäureanhydriden mit einem molekularen Sauerstoff enthaltenden Gas eingesetzt werden, verwendet.

Auch wenn die erfindungsgemäßen Multimetalloxide vorzugsweise für die Herstellung so genannter Schalenkatalysatoren eingesetzt werden, können sie auch als Vorläuferverbindung zur Herstellung herkömmlicher Trägerkatalysatoren oder von Volikatalysatoren, also Katalysatoren die kein Trägermaterial enthalten, verwendet werden.

Die Herstellung der erfindungsgemäßen Katalysatoren zur partiellen Oxidation aromatischer Kohlenwasserstoffe zu Aldehyden, Carbonsäuren und/oder Carbonsäureanhydriden aus den erfindungsgemäßen Multimetalloxiden erfolgt zweckmäßigerweise über die Stufe eines so genannten "Präkatalysators", der als solcher gelagert und gehandelt werden kann und aus dem der aktive Katalysator entweder durch thermische Behandlung hergestellt oder in situ im Oxidationsreaktor unter den Bedingungen der Oxidationsreaktion erzeugt werden kann. Bei dem Präkatalysator handelt es sich somit um eine Vorstufe des Katalysators, der aus einem inerten nicht-porösen Trägermaterial und wenigstens einer darauf schalenförmig aufgebrachten Schicht besteht, wobei diese Schicht vorzugsweise 30 bis 100 Gew.%, insbesondere 50 bis 100 Gew.-%, bezogen auf das Gesamtgewicht dieser Schicht, eines Multimetalloxids gemäß Formel Ienthalten. Besonders bevorzugt besteht die Schicht vollständig aus einem Multimetalloxid gemäß Formel I. enthält die katalytisch aktive Schicht außer dem Multimetalloxid gemäß Formel I noch weitere Komponenten, können dies z. B. Inertmaterialien, wie Siliciumcarbid oder Steatit, oder aber auch sonstige bekannte Katalysatoren zur Oxidation von aromatischen Kohlenwasserstoffen zu Aldehyden, Carbonsäuren und/oder Carbonsäureanhydriden auf Vanadiumoxid/Anatas-Basis sein. Vorzugsweise enthält der Präkatalysator 5 bis 25 Gew.%, bezogen auf das Gesamtgewicht des Präkatalysators, Multimetalloxid.

Als inertes nicht-poröses Trägermaterial für die erfindungsgemäßen Präkatalysatoren und Schalenkatalysatoren können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂) Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Wie erwähnt, können die vorstehend genannten Trägermaterialien in Pulverform auch der katalytisch aktiven Masse der erfindungsgemäßen Schalenkatalysatoren zugemischt werden.

Zur schalenförmigen Beschichtung des inerten Trägermaterials mit dem erfindungsgemäßen Multimetalloxid können im Prinzip bekannte Methoden des Standes der Technik angewandt werden. Beispielsweise kann die bei der Umsetzung des Vanadiumpentoxids mit einer Silberverbindung, einer Verbindung der Metallkomponente M und gegebenenfalls Q erhaltene Suspension gemäß den Verfahren von DE-A 16 92 938 und DE-A 17 69 998 in einer beheizten Dragiertrommel bei erhöhter Temperatur auf den aus inertem Trägermaterial bestehenden Katalysatorträger aufgesprüht werden, bis die gewünschte Menge an Multimetalloxid, bezogen auf das Gesamtgewicht des Präkatalysators erreicht ist. Anstelle von Dragiertrommeln können analog zu DE-A 21 06 796 auch Wirbelbettbeschichter, wie sie in DE-A 12 80 756 beschrieben sind, zur schalenförmigen Aufbringung des erfindungsgemäßen Multimetalloxids auf den Katalysatorträger eingesetzt werden. Anstelle der erhaltenen Suspension des erfindungsgemäßen Multimetalloxids, kann, besonders bevorzugt, eine Aufschlämmung des nach Isolierung und Trocknung erhaltenen Pulvers des erfindungsgemäßen Multimetalloxids bei diesen Beschichtungsverfahren verwendet werden. Analog EP-A 744 214 können der Suspension des erfindungsgemäßen Multimetalloxids, wie sie bei dessen Herstellung entsteht, oder einer Aufschlämmung eines Pulvers des erfindungsgemäßen, getrockneten Multimetalloxids in Wasser, einem organischen Lösungsmittel, wie höheren Alkoholen, mehrwertigen Alkoholen, z. B. Ethylenglykol, 1,4-Butandiol oder Glycerin, Dimethylformamid, Dimethylacetamid, Dimethylsulfoxid, N-Methylpyrrolidon oder cyclischen Harnstoffen, wie N,N'-Dimethylethylenharnstoff oder N,N'-Dimethylpropylenharnstoff, oder in Mischungen dieser organischen Lösungsmittel mit Wasser, organische Bindemittel, bevorzugt Copolymere, gelöst oder vorteilhaft in Form einer wässrigen Dispersion zugesetzt werden, wobei im Allgemeinen Bindemittelgehalte von 10 bis 20 Gew.%, bezogen auf den Feststoffgehalt der Suspension oder Aufschlämmung des erfindungsgemäßen Multimetalloxids angewandt werden. Geeignete Bindemittel sind z. B. Vinylacetat/Vinyllaurat-, Vinylacetat/Acrylat-, Styrol/Acrylat-, Vinylacetat/Maleat- oder Vinylacetat/Ethylen-Copolymere. Werden als Bindemittel organische Copolymer-Polyester, z. B. auf Basis von Acrylat/Dicarbonsäureanhydrid/Alkanolamin, in einer Lösung in einem organischen Lösungsmittel der Aufschlämmung des erfindungsgemäßen Multimetalloxids zugesetzt, kann analog zur Lehre der DE-A 198 23 262.4 der Gehalt an Bindemittel auf 1 bis 10 Gew.%, bezogen auf den Feststoffgehalt der Suspension oder Aufschlämmung, verringert werden.

Bei der Beschichtung des Katalysatorträgers mit den erfindungsgemäßen Multimetalloxiden werden im Allgemeinen Beschichtungstemperaturen von 20 bis 500 °C angewandt, wobei die Beschichtung in der Beschichtungsapparatur unter Atmosphärendruck oder unter reduziertem Druck erfolgen kann. Zur Herstellung der erfindungsgemäßen Präkatalysatoren wird die Beschichtung im Allgemeinen bei 0 °C bis 200 °C, vorzugsweise bei 20 bis 150 °C, insbesondere bei Raumtemperatur bis 100 °C durchgeführt. Bei der Beschichtung des Katalysatorträgers mit einer feuchten Suspension der erfindungsgemäßen Multimetalloxide kann es zweckmäßig sein, höhere Beschichtungstemperaturen, z. B. Temperaturen von 200 bis 500 °C, anzuwenden. Bei den vorstehend genannten tieferen Temperaturen kann bei Verwendung eines polymeren Bindemittels bei der Beschichtung ein Teil des Bindemittels in der auf dem Katalysatorträger aufgetragenen Schicht verbleiben.

Bei einer späteren Umwandlung des Präkatalysators in einen erfindungsgemäßen Schalenkatalysator durch thermische Behandlung bei Temperaturen über 200 bis 500 °C entweicht das Bindemittel durch thermische Zersetzung und/oder Verbrennung aus der aufgetragenen Schicht. Die Umwandlung des Präkatalysators in einen erfindungsgemäßen Schalenkatalysator kann auch durch thermische Behandlung bei Temperaturen über 500 °C erfolgen, beispielsweise bei Temperaturen bis 650 °C, vorzugsweise wird die thermische Behandlung bei Temperaturen von über 200 bis 500 °C, insbesondere bei 300 bis 450 °C durchgeführt.

Oberhalb 200 °C, insbesondere bei Temperaturen von mehr als 300 °C, zersetzen sich die erfindungsgemäßen Multimetalloxide unter Ausbildung von katalytisch aktiven Silber-Vanadiumoxid-Bronzen.

Unter Silber-Vanadiumoxid-Bronzen werden Silber-Vanadiumoxid-Verbindungen mit einem atomaren Ag : V-Verhältnis von weniger als 1 verstanden. Es handelt sich im Allgemeinen um halbleitende oder metallisch leitfähige, oxidische Festkörper, die bevorzugt in Schicht- oder Tunnelstrukturen kristallisieren, wobei das Vanadium im [V₂O₅]-Wirtsgitter teilweise reduziert zu V(IV) vorliegt.

Bei entsprechend hohen Beschichtungstemperaturen kann bereits ein Teil der auf den Katalysatorträger aufgetragenen Multimetalloxide zu katalytisch aktiven Silber-Vanadiumoxid-Bronzen und/oder bezüglich ihrer Struktur kristallographisch nicht aufgeklärten Silber-Vanadiumoxid-Verbindungen, die in die genannten Silber-Vanadiumoxid-Bronzen umgewandelt werden können, zersetzt werden. Bei Beschichtungstemperaturen von 300 bis 500 °C läuft diese Zersetzung praktisch vollständig ab, so dass bei einer Beschichtung bei 300 bis 500 °C der erfindungsgemäße Schalenkatalysator ohne Durchlaufen der Vorstufe des Präkatalysators erhalten werden kann.

Die erfindungsgemäßen Schalenkatalysatoren werden bevorzugt aus den erfindungsgemäßen Präkatalysatoren hergestellt oder aus diesen Präkatalysatoren im Reaktor für die Oxidation der aromatischen Kohlenwasserstoffe in situ erzeugt.

Bei der thermischen Behandlung der erfindungsgemäßen Präkatalysatoren bei Temperaturen von über 200 bis 650 °C, vorzugsweise bei über 250 bis 500 °C, insbesondere bei 300 bis 450 °C, zersetzen sich die im Präkatalysator enthaltenen Multimetalloxide zu Silber-Vanadiumoxid-Bronzen. Diese Umwandlung der im Präkatalysator enthaltenen erfindungsgemäßen Multimetalloxide zu Silber-Vanadiumoxid-Bronzen findet insbesondere auch in situ im Reaktor zur Gasphasenpartialoxidation von aromatischen Kohlenwasserstoffen zu Aldehyden, Carbonsäuren und/oder Carbonsäureanhydriden, beispielsweise im Reaktor zur Herstellung von Phthalsäureanhydrid aus o-Xylol und/oder Naphthalin, bei den dabei im Allgemeinen angewandten Temperaturen von 300 bis 450 °C statt, wenn man anstelle des erfindungsgemäßen Schalenkatalysators einen erfindungsgemäßen Präkatalysator bei dieser Umsetzung einsetzt. Bis zum Ende der Umwandlung des erfindungsgemäßen Multimetalloxids zu den Silber-Vanadiumoxid-Bronzen ist dabei in der Regel ein steter Anstieg der Selektivität des Schalenkatalysators zu beobachten. Die dabei entstehenden Silber-Vanadiumoxid-Bronzen sind somit ein katalytisch aktiver Bestandteil der katalytisch aktiven Schicht des erfindungsgemäßen Schalenkatalysators.

Die thermische Umwandlung der erfindungsgemäßen Multimetalloxide zu Silber-Vanadiumoxid-Bronzen verläuft über eine Reihe von Reduktions- und Oxidationsreaktionen, die im Einzelnen noch nicht verstanden sind.

Eine andere Möglichkeit zur Herstellung eines erfindungsgemäßen Schalenkatalysators besteht in der thermischen Behandlung des erfindungsgemäßen Multimetalloxidpulvers bei Temperaturen von oberhalb 200 bis 650 °C und der Beschichtung des inerten nicht-porösen Katalysatorträgers, gegebenenfalls unter Zusatz eines Bindemittels, mit der hierbei erhaltenen Silber-Vanadiumoxid-Bronze.

Besonders bevorzugt werden die erfindungsgemäßen Schalenkatalysatoren allerdings aus den erfindungsgemäßen Präkatalysatoren einstufig oder gegebenenfalls, nach einer thermischen Behandlung im Zuge oder nach der Beschichtung des Katalysatorträgers, mehrstufig, insbesondere einstufig, jeweils in situ im Oxidationsreaktor unter den Bedingungen der Oxidation von aromatischen Kohlenwasserstoffen zu Aldehyden, Carbonsäuren und/oder Carbonsäureanhydriden, erzeugt.

Die katalytisch aktive Schale des erfindungsgemäß hergestellten Schalenkatalysators enthält im Allgemeinen 30 bis 100 Gew.%, vorzugsweise 50 bis 100 Gew.%, bezogen auf das Gesamtgewicht der katalytisch aktiven Schale, der so erzeugten Silber-Vanadiumoxid-Bronzen, wobei das Silber und das Vanadium in der katalytisch aktiven Schale im Allgemeinen in einem Atomverhältnis Ag : V von 0,15 bis 0,95, vorzugsweise von 0,25 bis 0,5 und besonders bevorzugt von 0,3 bis 0,45 vorliegen. Besonders bevorzugt besteht die katalytisch aktive Schicht der erfindungsgemäßen Schalenkatalysatoren vollständig aus den erfindungsgemäß erzeugten Silber-Vanadiumoxid-Bronzen. Enthält die katalytisch aktive Schicht oder Schichten außer den erfindungsgemäß erzeugten Silber-Vanadiumoxid-Bronzen noch weitere Komponenten, können dies z. B. Inertmaterialien des Standes der Technik, wie Siliciumcarbid oder Steatit, sein oder aber auch nicht-erfindungsgemäße Katalysatorverbindungen zur Oxidation von aromatischen Kohlenwasserstoffen zu Aldehyden, Carbonsäuren und/oder Carbonsäureanhydriden z. B. auf Vanadiumpentoxid/Anatas-Basis, wie sie eingangs beispielhaft bei der Schilderung des Standes der Technik erwähnt wurden. Die Schichtdicke der die katalytisch aktiven Bestandteile enthaltenden Katalysatorschale beträgt im Allgemeinen 10 bis 250 mm. Dies gilt auch, falls die Katalysatorschale aus mehreren, nacheinander aufgetragenen Schichten besteht.

Die BET-Oberfläche der erfindungsgemäßen Schalenkatalysatoren beträgt im Allgemeinen 2 bis 100 m²/g, vorzugsweise 2 bis 40 m²/g und besonders bevorzugt 3 bis 20 m²/g_{.}

Die erfindungsgemäßen Schalenkatalysatoren werden für die partielle Oxidation von aromatischen Kohlenwasserstoffen zu Aldehyden, Carbonsäuren und/oder Carbonsäureanhydriden, insbesondere zur Gasphasenpartialoxidation von o-Xylol und/oder Naphthalin zu Phthalsäureanhydrid oder von Toluol zu Benzoesäure und/oder Benzaldehyd, mit einem molekularen Sauerstoff enthaltenden Gas verwendet. Die erfindungsgemäßen Katalysatoren können zu diesem Zweck alleine oder in Kombination mit anderen, unterschiedlich aktiven Katalysatoren, beispielsweise Katalysatoren des Standes der Technik auf Vanadiumoxid/Anatas-Basis, eingesetzt werden, wobei die unterschiedlichen Katalysatoren im Allgemeinen in separaten Katalysatorschüttungen, die in einem oder mehreren Katalysatorfestbetten angeordnet sein können, im Reaktor angeordnet werden.

Die erfindungsgemäßen Schalenkatalysatoren oder Präkatalysatoren werden hierzu in die Reaktionsrohre eines Röhrenreaktors gefüllt, die von außen, z. B. mittels einer Salzschmelze, auf die Reaktionstemperatur thermostatisiert werden. Wird anstelle des erfindungsgemäßen Schalenkatalysators ein erfindungsgemäßer Präkatalysator eingesetzt, entsteht daraus unter den Temperaturbedingungen der partiellen Oxidation von aromatischen Kohlenwasserstoffen zu Aldehyden, Carbonsäuren und/oder Carbonsäureanhydriden, insbesondere bei der Partialoxidation von *o*-Xylol und/oder Naphthalin zu Phthalsäureanhydrid oder bei der Partialoxidation von Toluol zu Benzoesäure und Benzaldehyd, ein erfindungsgemäßer Schalenkatalysator. Über die so bereitete Katalysatorschüttung wird das Reaktionsgas bei Temperaturen von 100 bis 650 °C und vorzugsweise 250 bis 480 °C und bei einem Überdruck von im Allgemeinen 0,1 bis 2,5 bar, vorzugsweise von 0,3 bis 1,5 bar mit einer Raumgeschwindigkeit von im Allgemeinen 750 bis 5000 h⁻¹ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel, wie Wasserdampf, Kohlendioxid und/oder Stickstoff, enthalten kann, mit dem zu oxidierenden, aromatischen Kohlenwasserstoff erzeugt, wobei das molekularen Sauerstoff enthaltende Gas im Allgemeinen 1 bis 100 Vol.-%, vorzugsweise 2 bis 50 Vol.% und besonders bevorzugt 10 bis 30 Vol.-% Sauerstoff, 0 bis 30 Vol.-%, vorzugsweise 0 bis 20 Vol.% Wasserdampf sowie 0 bis 50 Vol.%, vorzugsweise 0 bis 1 Vol.% Kohlendioxid, Rest Stickstoff, enthalten kann. Zur Erzeugung des Reaktionsgases wird das molekularen Sauerstoff enthaltende Gas im Allgemeinen mit 30 bis 300 g je Nm³, bevorzugt mit 70 bis 150 g je Nm³ Gas des zu oxidierenden aromatischen Kohlenwasserstoffs beschickt.

Besonders vorteilhaft wird als molekularen Sauerstoff enthaltendes Gas Luft verwendet

Vorteilhaft wird die Gasphasenpartialoxidation so durchgeführt, dass man zwei oder mehr Zonen, vorzugsweise zwei Zonen, der im Reaktionsrohr befindlichen Katalysatorschüttung auf unterschiedliche Reaktionstemperaturen thermostatisiert, wozu beispielsweise Reaktoren mit getrennten Salzbädern, wie sie in DE-A 22 01 528 oder DE-A 28 30 765 beschrieben sind, eingesetzt werden können. Wird die Umsetzung in zwei Reaktionszonen durchgeführt, wie in DE-A 40 13 051 beschrieben, wird im Allgemeinen die zum Gaseintritt des Reaktionsgases hin gelegene Reaktionszone, welche im Allgemeinen 30 bis 80 Vol.% des gesamten Katalysatorvolumens umfasst, auf eine um 1 bis 20 °C, vorzugsweise um 1 bis 10 °C und insbesondere um 2 bis 8 °C höhere Reaktionstemperatur als die zum Gasaustritt hin gelegene Reaktionszone thermostatisiert. Eine solche Arbeitsweise wird als Zwei- oder Mehrzonenstrukturierung des Reaktors bezeichnet. Alternativ kann die Gasphasenoxidation auch ohne Aufteilung in Temperaturzonen bei einer einheitlichen Reaktionstemperatur durchgeführt werden.

Bei einer bevorzugten Ausführungsform des Verfahrens zur partiellen Oxidation von aromatischen Kohlenwasserstoffen zu Aldehyden, Carbonsäuren und/oder Carbonsäureanhydriden, die sich besonders vorteilhaft für die Herstellung von Phthalsäureanhydrid aus o-Xylol und/oder Naphthalin erweist, wird der aromatische Kohlenwasserstoff zunächst an einer Schüttung des erfindungsgemäßen Schalenkatalysators unter Teilumsatz zu einem Reaktionsgemisch umgesetzt. Das erhaltene Reaktionsgemisch oder eine Fraktion davon kann man dann mit wenigstens einem weiteren Katalysator in Kontakt bringen, dessen katalytisch aktive Masse Vanadiumpentoxid und Anatas enthält.

Vorzugsweise leitet man den gasförmigen Strom nacheinander über ein Bett eines stromaufwärts gelegenen Katalysators und ein Bett eines stromabwärts gelegenen Katalysators leitet, wobei das Bett des stromaufwärts gelegenen Katalysators einen erfindungsgemäßen Katalysator enthält und das Bett des stromabwärts gelegenen Katalysators wenigstens einen Katalysator enthält, dessen katalytisch aktive Masse Vanadiumpentoxid und Anatas enthält. Im Allgemeinen enthält die katalytisch aktive Masse des stromabwärts gelegenen Katalysators 1 bis 40 Gew.% Vanadiumoxid, berechnet als V₂O₅, 60 bis 99 Gew.-% Titandioxid, berechnet als TiO₂, bis zu 1 Gew.-% einer Cäsiumverbindung, berechnet als Cs, bis zu 1 Gew.-% einer Phosphorverbindung, berechnet als P, und bis zu 10 Gew.% Antimonoxid, berechnet als Sb₂O₃. Mit Vorteil umfasst das Bett des stromabwärts gelegenen Katalysators wenigstens zwei Lagen von Katalysatoren, deren katalytisch aktive Masse unterschiedlichen Cs-Gehalt aufweist, wobei der Cs-Gehalt im Strömungsrichtung des gasförmigen Stroms abnimmt.

Im Fall der Herstellung von Phthalsäureanhydrid aus o-Xylol enthält das teilumgesetzte Reaktionsgemisch z. B. Phthalsäureanhydrid und andere Oxidationsprodukte, wie o-Tolylaldehyd, o-Tolylcarbonsäure und Phthalid, und nicht umgesetztes o-Xylol. Es kann dann weiterverarbeitet werden, indem entweder
a) das o-Xylol vom Phthalsäureanhydrid und den anderen Oxidationsprodukten, die Intermediate auf dem Reaktionsweg von o-Xylol zu Phthalsäureanhydrid sind, abgetrennt und zurückgeführt wird und der Strom aus Phthalsäureanhydrid und Intermediaten einer oder mehreren weiteren Katalysatorschüttungen mit z. B. einem Schalenkatalysator auf Vanadiumoxid/Anatas-Basis zugeführt wird, wo die Intermediate selektiv zu Phthalsäureanhydrid oxidiert werden; oder indem
b) das Produktgemisch ohne weitere Aufarbeitung, d. h. ohne o-Xylol-Abtrennung, über eine zweite oder gegebenenfalls über weitere Katalysatorschüttungen geleitet wird.

Durch diese Art der Reaktionsführung wird insgesamt eine deutlich höhere Phthalsäureanhydrid-Ausbeute erzielt als bei alleiniger Verwendung von Katalysatorsystemen auf Vanadiumoxid/Anatas-Basis, da die erfindungsgemäßen Schalenkatalysatoren o-Xylol und/oder Naphthalin wesentlich selektiver zu Phthalsäureanhydrid bzw. den vorstehend genannten Intermediaten oxidieren können.

Auf analoge Weise kann bei der Oxidation von Toluol zu Benzaldehyd und/oder Benzoesäure verfahren werden. Benzaldehyd findet beispielsweise als Aromastoff Verwendung.

### Beispiele

### A Katalysatoren

### A.1 Ce_{0,02}Ag_{0.71}V₂Oₓ (erfindungsgemäßer Katalysator)

In 7 I vollentsalztes Wasser von 60 °C wurden 102 g V₂O₅ (= 0,56 Mol) unter Rühren zugegeben. Die Suspension wurde mit eine wässrigen Lösung von 4,94 g CeNO₃* 6 H₂O (= 0,011 Mol, Aldrich, Reinheit 99 %) versetzt. In die erhaltene orangefarbene Suspension wurde unter weiterem Rühren eine wässrige Lösung von 68 g AgNO₃ (= 0,398 Mol) in 1l Wasser zugegeben. Anschließend wurde die Temperatur der erhaltenen Suspension innerhalb von 2 Stunden auf 90 °C erhöht und bei dieser Temperatur die Mischung 24 Stunden gerührt. Danach wurde die erhaltene dunkelbraune Suspension abgekühlt und sprühgetrocknet (Eingangstemperatur (Luft) = 350 °C, Ausgangstemperatur (Luft) = 110 °C).

Das erhaltene Pulver hatte eine spezifische Oberfläche nach BET von 61 m²/g_{.} Vom erhaltenen Pulver wurde ein Pulverröntgendiagramm mit Hilfe eines Diffraktometers D 5000 der Firma Siemens unter Anwendung von Cu-Kα-Strahlung (40 kV, 30 mA) aufgenommen. Das Diffraktometer war mit einem automatischen Primär- und Sekundärblendensystem sowie einm Sekundär-Monochromator und Szintillationsdetektor ausgestattet. Aus dem Pulverröntgendiagramm wurden die folgenden Netzebenenabstände d [Å] mit den dazugehörigen relativen Intensitäten Iᵣₑₗ [%] erhalten: 15,04 (11,9), 11,99 (8,5), 10,66 (15,1), 5,05 (12,5), 4,35 (23), 3,85 (16,9), 3,41 (62,6), 3,09 (55,1), 3,02 (100), 2,58 (23,8), 2,48 (27,7), 2,42 (25,1), 2,36 (34,2), 2,04 (26,4), 1,93 (33,2), 1,80 (35,1), 1,55 (37,8).

### A.2 Mn_{0,02}Ag_{0,71}V₂Oₓ (erfindungsgemäßer Katalysator)

In 7 I vollentsalztes Wasser von 60 °C wurden 102 g V₂O₅ (= 0,56 Mol) unter Rühren zugegeben. Die Suspension wurde mit eine wässrigen Lösung von 2,76 g Mn(NO₃)₂ * 4 H₂O (= 0,011 Mol, Chempur, Reinheit 98,5 %) versetzt. In die erhaltene orangefarbene Suspension wurde unter weiterem Rühren eine wässrige Lösung von 68 g AgNO₃ (= 0,398 Mol) in 1 I Wasser zugegeben. Anschließend wurde die Temperatur der erhaltenen Suspension innerhalb von 2 Stunden auf 90 °C erhöht und bei dieser Temperatur die Mischung 24 Stunden gerührt. Danach wurde die erhaltene dunkelbraune Suspension abgekühlt und sprühgetrocknet (Eingangstemperatur (Luft) = 350 °C, Ausgangstemperatur (Luft) = 110 _{°}C)_{.}

Das erhaltene Pulver hatte eine spezifische Oberfläche nach BET von 58 m²/g. Vom erhaltenen Pulver wurde ein Pulverröntgendiagramm aufgenommen. Aus dem Pulverröntgendiagramm wurden die folgenden Netzebenenabstände d [Å] mit den dazugehörigen relativen Intensitäten Iᵣₑₗ, [%] erhalten: 15,09 (6,8), 11,98 (5,7), 10,61 (9,4), 4,36 (16,8), 3,84 (14,7), 3,40 (81,7), 3,09 (61.1), 3,01 (100), 2,58 (26,4), 2,47 (27,9), 2,41 (21,6), 2,36 (37,8), 2,04 (32,2), 1,93 (28,9), 1,80 (42,2), 1,55 (43,4).

### A.3 Ag_{0,73}V₂Ox (Vergleichskatalysator)

In 7 l vollentsalztes Wasser von 60 °C wurden 102 g V₂O₅ (= 0,56 Mol) unter Rühren zugegeben. In die erhaltene orangefarbene Suspension wurde unter weiterem Rühren eine wässrige Lösung von 69,5 g AgNO₃ (= 0,409 Mol) in 1 I Wasser zugegeben. Anschließend wurde die Temperatur der erhaltenen Suspension innerhalb von 2 Stunden auf 90 °C erhöht und bei dieser Temperatur die Mischung 24 Stunden gerührt. Danach wurde die erhaltene dunkelbraune Suspension abgekühlt und sprühgetrocknet (Eingangstemperatur (Luft) = 350 °C, Ausgangstemperatur (Luft) = 110 °C). Das erhaltene Pulver hatte eine spezifische Oberfläche nach BET von 56 m²/g_{.}

Für das nachstehende Beispiel B.1 wurden die so hergestellten Pulver wie folgt auf Magnesiumsilikat-Kugeln aufgebracht: 300 g Steatit-Kugeln mit einem Durchmesser von 3,5 bis 4 mm wurden in einer Dragiertrommel bei 20 °C während 20 min mit 40 g des Pulvers und 4,4 g Oxalsäure unter Zusatz von 35,3 g eines 60 Gew.% Wasser und 40 Gew.% Glycerin enthaltenden Gemisches beschichtet und anschließend getrocknet. Das Gewicht der so aufgetragenen katalytisch aktiven Masse, bestimmt an einer Probe des erhaltenen Präkatalysators, betrug nach einstündiger Wärmebehandlung bei 400 °C 10 Gew.%, bezogen auf das Gesamtgewicht des fertigen Katalysators.

Für die Beispiele B.2 bis B.4 wurden die Pulver wie folgt auf Magnesiumsilikat-Ringe aufgebracht: 350 g Steatit-Ringe mit einem äußeren Durchmesser von 7 mm, einer Länge von 3 mm und einer Wandstärke von 1,5 mm wurden in einer Dragiertrommel bei 20 °C während 20 min mit 84,4 g des Pulvers und 9,4 g Oxalsäure unter Zusatz von 66,7 g eines 60 Gew.% Wasser und 40 Gew.% Glycerin enthaltenden Gemisches beschichtet und anschließend getrocknet. Das Gewicht der so aufgetragenen katalytisch aktiven Masse, bestimmt an einer Probe des erhaltenen Präkatalysators, betrug nach einstündiger Wärmebehandlung bei 450 °C 18 Gew.%, bezogen auf das Gesamtgewicht des fertigen Katalysators.

### A.4 Referenzkatalysator (V₂O₅/TiO₂-Zweilagenkatalysator)

1400 g Steatit (Magnesiumsilikat) Ringe mit einem äußeren Durchmesser von 8 mm, einer Länge von 6 mm und einer Wandstärke von 1,6 mm wurden in einer Dragiertrommel auf 160 °C erhitzt und zusammen mit 13,8 g eines organischen Binders, bestehend aus einem Copolymer von Acrylsäure/Maleinsäure (Gewichtsverhältnis 75:25), mit einer Suspension aus 466 g Anatas mit einer BET-Oberfläche von 21 m²/g, 67,2 g Vanadyloxalat, 14,4 g Antimontrioxid, 3,15 g Ammoniumhydrogenphosphat, 2,87 g Cäsiumsulfat, 721 g Wasser und 149 g Formamid besprüht. Die auf diese Weise aufgebrachte katalytisch aktive Masse bestand im Mittel aus 0,16 Gew.% Phosphor (berechnet als P), 7,5 Gew.% Vanadium (berechnet als V₂O₅), 3,2 Gew.-% Antimon (berechnet als Sb₂O₃), 0,40 Gew.% Cäsium (berechnet als Cs) und 88,74 Gew.-% Titandioxid.

Der so erhaltene Schalenkatalysator wurde in einer Dragiertrommel auf 160 C erhitzt und zusammen mit 14 g eines organischen Binders, bestehend aus einem Copolymer von Acrylsäure/Maleinsäure (Gewichtsverhältnis 75:25), mit einer Suspension aus 502 g Anatas mit einer BET-Oberfläche von 21 m²/g, 35,8 g Vanadyloxalat, 2,87 g Cäsiumsulfat, 720 g Wasser und 198 g Formamid besprüht. Die auf diese Weise aufgebrachte katalytisch aktive Masse bestand im Mittel aus 4,0 Gew.% Vanadium (berechnet als V₂O₅), 0,4 Gew.% Cäsium (berechnet als Cs) und 88,8 Gew.% Titandioxid. Das Gewicht der aufgetragenen Schichten betrug 9,3 Gew.% des Gesamtgewichts des fertigen Katalysators.

### A.5 Referenzkatalysator (V₂O₅/TiO₂-Katalysator)

1400 g Steatit (Magnesiumsilikat) Ringe mit einem äußeren Durchmesser von 8 mm, einer Länge von 6 mm und einer Wandstärke von 1,6 mm wurden in einer Dragiertrommel auf 160 °C erhitzt und mit einer Suspension aus 468 g Anatas mit einer BET-Oberfläche von 21 m²/g, 67,2 g Vanadyloxalat, 16,8 g Antimontrioxid, 2,95 g Ammoniumhydrogenphosphat, 0,72 g Cäsiumsulfat, 719 g Wasser und 150 g Formamid besprüht, bis das Gewicht der auf getragenen Schicht 10,5 % des Gesamtgewichts, des fertigen Katalysators betrug (nach einstündiger Wärmebehandlung bei 450 °C). Die auf diese Weise aufgebrachte katalytisch aktive Masse, also die Katalysatorschale, bestand im Mittel aus 0,15 Gew.-% Phosphor (berechnet als P), 7,5 Gew.-% Vanadium (berechnet als V₂O₅), 3,2 Gew.% Antimon (berechnet als Sb₂O₃), 0,1 Gew.-% Cäsium (berechnet als Cs) und 89,05 Gew.-% Titandioxid.

### B Oxidationen

### B.1 Herstellung von Phthalsäureanhydrid mit den erfindungsgemäßen Katalysatoren gemäß Beispiel A.1 und A.2 und dem Vergleichskatalysator gemäß Beispiel A.3

In ein 80 cm langes Eisenrohr mit einer lichten Weite von 16 mm wurden die Katalysatoren A.1, A.2 bzw. A.3 (beschichtete Steatit-Kugeln) bis zu einer Bettlänge von 66 cm eingefüllt. Das Eisenrohr war zur Temperaturregelung mit einem Elektroheizmantel umgeben. Durch das Rohr wurden stündlich von oben nach unten 360 NI-Luft mit einer Beladung an 98,5 gew.%igem o-Xylol von 60 g o-Xylol/Nm³ Luft geleitet. In der nachstehenden Tabelle sind die erhaltenen Ergebnisse zusammengefasst.

**Tabelle**

| Katalysator | Zusammensetzung der Aktivmasse | Reaktionstemperatur (°C) | Umsatz (%) | CO_{X}-Selektivität¹) (%) |
|---|---|---|---|---|
| Katalysator gemäß Beispiel A.1 | Ce_{0,02}Ag_{0.71}V₂Oₓ | 350 | 58 | 11 |
| Katalysator gemäß Beispiel A.2 | Mn_{0,02}Ag_{0,71}V₂Oₓ | 350 | 48 | 13 |
| Vergleichskatalysator gemäß Beispiel A.3 | Ag_{0,73}V₂Oₓ | 350 | 37 | 10 |

| | | | | |
|---|---|---|---|---|
| 1) "COₓ-Selektivität" entspricht dem Anteil des zu Verbrennungsprodukten (CO, CO₂) umgesetzten o-Xylols; die Restselektivität auf 100 % entspricht dem Anteil des zu dem Wertprodukt Phthalsäureanhydrid und den Zwischenprodukten o-Tolylaldehyd, o-Tolylsäure und Phthalid sowie Nebenprodukten wie Maleinsäureanhydrid, Citraconsäureanhydrid und Benzoesäure umgesetzten o-Xylols | | | | |

An einer Ausbauprobe des Katalysators A.1 wurde eine BET-Oberfläche der Aktivmasse von 6,7 m²/g und eine Vanadium-Oxidationsstufe von 4,63 ermittelt. Aus dem Pulverröntgendiagramm wurden die folgenden Netzebenenabstände d [Å] mit den dazugehörigen relativen Intensitäten Iᵣₑₗ [%] erhalten: 4,85 (9,8), 3,50 (14,8), 3,25 (39,9), 2,93 (100), 2,78 (36,2), 2,55 (35,3), 2,43 (18,6),1,97 (15,2),1,95 (28,1),1,86 (16,5), 1,83 (37,5), 1,52 (23,5).

### B.2 Herstellung von Phthalsäureanhydrid mit einer Kombination des Vergleichskatalysators gemäß Beispiel A.3 und den Referenzkatalysatoren A.4 und A.5 in einem Rohr

Von unten nach oben wurden jeweils 0,80 m des Katalysators A.5, 1,40 m des Katalysators A.4 und anschließend 0,80 m des Präkatalysators A.3 (beschichtete Steatit-Ringe) in ein 3,85 m langes Eisenrohr mit einer lichten Weite von 25 mm eingefüllt. Das Eisenrohr war zur Temperaturregelung von einer Salzschmelze umgeben. Durch das Rohr wurden stündlich von oben nach unten 4,0 Nm³ Luft mit einer Beladung an 98,5 gew.%igem o-Xylol von 80 g o-Xylol/Nm³ Luft geleitet. Dabei wurde bei einer Salzbadtemperatur von 353 bis 360 °C eine durchschnittliche PSA-Ausbeute von 115,5 Gew.-% erreicht (Ausbeute bedeutet das erhaltene Phthalsäureanhydrid in Gewichtsprozent, bezogen auf 100%iges o-Xylol). Der Umsatz betrug mehr als 99,94 %, der Rest-Phthalid-Gehalt am Reaktorausgang lag bei weniger als 0,35 Gew.-%.

### B.3 Herstellung von Phthalsäureanhydrid mit einer Kombination des erfindungsgemäßen Katalysators gemäß Beispiel A.1 und den Referenzkatalysatoren A.4 und A.5 in einem Rohr

Beispiel B.2 wurde wiederholt, wobei jedoch jeweils 0,80 m des Katalysators A.5, 1,40 m des Katalysators A.4 und anschließend 0,80 m des Präkatalysators A.1 eingefüllt wurden. Es wurde eine durchschnittliche PSA-Ausbeute von 117,2 Gew.-% erreicht.

### B.4 Herstellung von Phthalsäureanhydrid mit einer Kombination des erfindungsgemäßen Katalysators gemäß Beispiel A.2 und den Referenzkatalysatoren A.4 und A.5 in einem Rohr

Beispiel B.3 wurde wiederholt, wobei jedoch jeweils 0,90 m des Katalysators A.5, 1,60 m des Katalysators A.4 und anschließend 0,50 m des Präkatalysators A.1 eingefüllt wurden. Es wurde eine durchschnittliche PSA-Ausbeute von 116,7 Gew.% erreicht. Der Umsatz betrug mehr als 99,94 %, der Rest-Phthalid-Gehalt am Reaktorausgang lag bei weniger als 0,35 Gew.-%. Dieses Beispiel zeigt, dass bei Verwendung der erfindungsgemäßen Katalysatoren hohe PSA-Ausbeuten auch mit einer gegenüber dem Beispiel B.1 deutlich verkürzten Bettlänge des Silber-Vanadiumoxid-Katalysators erreicht werden können.

## Patentansprüche

1. Multimetalloxid der allgemeinen Formel I
Ag_{a-c}Q_{b}M_{c}V₂O_{d} * e H₂O, I
worin
a einen Wert von 0,6 bis 0,9 hat,
Q für ein unter P, As, Sb und/oder Bi ausgewähltes Element steht,
b einen Wert von 0 bis 0,3 hat,
M für ein unter Ce und/oder Mn ausgewähltes Metall steht,
c einen Wert von 0,01 bis 0,1 hat,
d eine Zahl, die sich durch die Wertigkeit und Häufigkeit der von Sauerstoff verschiedenen Elemente in der Formel I bestimmt, bedeutet und
e einen Wert von 0 bis 20 hat,
das in einer Kristallstruktur vorliegt, deren Pulverröntgendiagramm durch Beugungsreflexe bei den Netzebenenabständen d 15,23 ± 0,6, 12,16 ± 0,4, 10,68 ± 0,3, 3,41 ± 0,04, 3,09 ±0,04, 3,02 ± 0,04, 2,36 ± 0,04 und 1,80 ± 0,04 Å gekennzeichnet ist.

2. Multimetalloxid nach Anspruch 1, in dem
b den Wert 0 hat.

3. Multimetalloxid nach Anspruch 1 oder 2, mit einer spezifischen Oberfläche nach BET von 3 bis 250 m²/g_{.}

4. Multimetalloxid nach einem der Ansprüche 1 bis 3, in dem M für Ce oder Mn steht.

5. Verwendung eines Multimetalloxids nach einem der Ansprüche 1 bis 4 zur Herstellung von Präkatalysatoren und Katalysatoren für die Gasphasenpartialoxidation von aromatischen Kohlenwasserstoffen.

6. Präkatalysator, der in einen Katalysator zur Gasphasenpartialoxidation von aromatischen Kohlenwasserstoffen umwandelbar ist, bestehend aus einem inerten nicht-porösen Träger und wenigstens einer darauf aufgebrachten Schicht, die ein Multimetalloxid nach einem der Ansprüche 1 bis 4 umfasst.

7. Präkatalysator nach Anspruch 6, der 5 bis 25 Gew.-%, bezogen auf das Gesamtgewicht des Präkatalysators, Multimetalloxid enthält.

8. Präkatalysator nach Anspruch 6 oder 7, dessen inertes nicht-poröses Trägermaterial aus Steatit besteht.

9. Katalysator zur Gasphasenpartialoxidation von aromatischen Kohlenwasserstoffen, bestehend aus einem inerten nicht-porösen Träger und wenigstens einer darauf aufgebrachten Schicht, die als katalytisch aktive Masse eine Silber-Vanadiumoxid-Bronze umfasst, die wenigstens ein unter Ce und/oder Mn ausgewähltes Metall M enthält, herstellbar aus einer Multimetalloxidmasse nach Anspruch 1 oder einem Präkatalysator nach Anspruch 6 durch thermische Behandlung bei Temperaturen oberhalb 200 °C bis 650 °C.

10. Katalysator nach Anspruch 9, wobei die Silber-Vanadium-Bronze Ce oder Mn enthält.

11. Katalysator nach Anspruch 9 oder 10 mit einer Schicht, deren katalytisch aktive Masse eine BET-Oberfläche von 2 bis 100 m²/g hat.

12. Verfahren zur Herstellung von Aldehyden, Carbonsäuren und/oder Carbonsäureanhydriden, bei dem man einen gasförmigen Strom, der einen aromatischen Kohlenwasserstoff und ein molekularen Sauerstoff enthaltendes Gas umfasst, bei erhöhter Temperatur mit einem Katalysator nach einem der Ansprüche 9 bis 11 in Kontakt bringt.

13. Verfahren nach Anspruch 12, bei dem man den Katalysator in situ aus einem Präkatalysator nach einem der Ansprüche 6 bis 8 erzeugt.

14. Verfahren nach Anspruch 12 oder 13, bei dem man das erhaltene Reaktionsgemisch oder eine Fraktion davon mit wenigstens einem weiteren Katalysator in Kontakt bringt, dessen katalytisch aktive Masse Vanadiumpentoxid und Anatas enthält.

15. Verfahren nach Anspruch 14, bei dem man den gasförmigen Strom nacheinander über ein Bett eines stromaufwärts gelegenen Katalysators und ein Bett eines stromabwärts gelegenen Katalysators leitet, wobei das Bett des stromaufwärts gelegenen Katalysators einen Katalysator nach Anspruch 9 enthält und das Bett des stromabwärts gelegenen Katalysators wenigstens einen Katalysator enthält, dessen katalytisch aktive Masse Vanadiumpentoxid und Anatas enthält.

16. Verfahren nach Anspruch 15, bei dem die katalytisch aktive Masse des stromabwärts gelegenen Katalysators 1 bis 40 Gew.-% Vanadiumoxid, berechnet als V₂O₅, 60 bis 99 Gew.-% Titandioxid, berechnet als TiO₂, bis zu 1 Gew.-% einer Cäsiumverbindung, berechnet als Cs, bis zu 1 Gew.-% einer Phosphorverbindung, berechnet als P, und bis zu 10 Gew.-% Antimonoxid, berechnet als Sb₂O₃, enthält.

17. Verfahren nach Anspruch 16, bei dem das Bett des stromabwärts gelegenen Katalysators wenigstens zwei Lagen von Katalysatoren umfasst, deren katalytisch aktive Masse unterschiedlichen Cs-Gehalt aufweist, wobei der Cs-Gehalt im Strömungsrichtung des gasförmigen Stroms abnimmt.

18. Verfahren nach einem der Ansprüche 12 bis 17, bei dem man als aromatischen Kohlenwasserstoff o-Xylol oder Naphthalin oder Mischungen aus o-Xylol und Naphthalin zu Phthalsäureanhydrid oxidiert.

## Claims

1. A multimetal oxide of the formula I,
Ag_{a-c}Q_{b}M_{c}V₂O_{d} * e H₂O, I
where
a is from 0.6 to 0.9,
Q is an element selected from among P, As, Sb and/or Bi,
b is from 0 to 0.3,
M is a metal selected from among Ce and Mn,
c is from 0.01 to 0.1,
d is a number which is determined by the valence and abundance of the elements other than oxygen in the formula I and
e is from 0 to 20,
which has a crystal structure whose X-ray powder diffraction pattern displays reflections at lattice plane spacings d of 15.23 ± 0.6, 12.16 ± 0.4, 10.68 ± 0.3, 3.41 ± 0.04, 3.09 ±0.04, 3.02 ± 0.04, 2.36 ± 0.04 and 1.80 ± 0.04 Å.

2. The multimetal oxide according to claim 1 in which
b is 0.

3. The multimetal oxide according to claim 1 or 2 which has a specific surface area determined by the BET method of from 3 to 250 m²/g.

4. The multimetal oxide according to any of claims 1 to 3 in which M is Ce or Mn.

5. The use of a multimetal oxide according to any of claims 1 to 4 for producing precatalysts and catalysts for the gas-phase partial Oxidation of aromatic hydrocarbons.

6. A precatalyst which can be converted into a catalyst for the gas-phase partial Oxidation of aromatic hydrocarbon and comprises an inert nonporous support and at least one layer comprising a multimetal oxide according to any of claims 1 to 4 applied thereto.

7. The precatalyst according to claim 6 which comprises from 5 to 25% by weight, based on the total weight of the precatalyst, of multimetal oxide.

8. The precatalyst according to claim 6 or °C whose inert nonporous support material comprises steatite.

9. A catalyst for the gas-phase partial Oxidation of aromatic hydrocarbons which comprises an inert nonporous support and, applied thereto, at least one layer comprising, as catalytically active composition, a silver-vanadium oxide bronze which comprises at least one metal M selected from among Ce and Mn, which catalyst can be produced from a multimetal oxide composition according to claim 1 or a precatalyst according to claim 6 by thermal treatment at temperatures of from >200°C to 650°C.

10. The catalyst according to claim 9, wherein the silver-vanadium bronze contains Ce or Mn.

11. The catalyst according to claim 9 or 10 having a layer whose catalytically active composition has a BET surface area of from 2 to 100 m²/g.

12. A process for preparing aldehydes, carboxylic acids and/or carboxylic anhydrides, in which a gaseous stream which comprises an aromatic hydrocarbon and a gas comprising molecular oxygen is brought info contact with a catalyst according to any of claims 9 to 11 at elevated temperature.

13. The process according to claim 12, wherein the catalyst is produced in situ from a precatalyst according to any of claim 6 to 8.

14. The process according to claim 12 or 13, wherein the reaction mixture obtained or a fraction thereof is brought into contact with at least one further catalyst whose catalytically active composition comprises vanadium pentoxide and anatase.

15. The process according to claim 14, wherein the gaseous stream is passed successively over a bed of an upstream catalyst and a bed of a downstream catalyst, where the bed of upstream catalyst comprises a catalyst according to claim 9 and the bed of downstream catalyst comprises at least one catalyst whose catalytically active composition comprises Vanadium pentoxide and anatase.

16. The process according to claim 15, wherein the catalytically active composition of the downstream catalyst comprises from 1 to 40% by weight of Vanadium oxide, calculated as V₂O₅, from 60 to 99% by weight of titanium dioxide, calculated as TiO₂, up to 1% by weight of a cesium compound, calculated is Cs, up to 1% by weight of a phosphorus compound, calculated as P, and up to 10% by weight of antimony oxide, calculated as Sb₂O₃.

17. The process according to claim 16, wherein the bed of the downstream catalyst comprises at least two layers of catalysts whose catalytically active composition has a differing Cs content, with the Cs content decreasing in the flow direction of the gaseous stream.

18. The process according to any of claims 12 to 17, wherein o-xylene or naphthalene or a mixture of o-xylene and naphthalene is used is aromatic hydrocarbon and is oxidized to phthalic anhydride.

## Revendications

1. Oxyde multimétallique de formule générale 1
Ag_{a-c}Q_{b}M_{c}V₂O_{d} * e H₂O, I
où
a a une valeur de 0,6 à 0,9,
Q représente un élément choisi parmi P, As, Sb et/ou Bi,
b a une valeur de 0 à 0,3,
M représente un métal choisi parmi Ce et/ou Mn,
c a une valeur de 0,01 à 0,1,
d vaut un nombre qui est déterminé par la valence et la fréquence des éléments différents de l'oxygène dans la formule générale I et
e a une valeur de 0 à 20,
qui se trouve dans une structure cristalline dont le digramme de diffraction de rayons X réalisé sur une poudre est **caractérisé par** des réflexes de diffraction aux distances entre les plans de réseau d de 15,23 ± 0,6, 12,16 ± 0,4, 10,68 ± 0,3, 3,41 ± 0,04, 3,09 ± 0,04, 3,02 ± 0,04, 2,36 ± 0,04 et 1,80 ± 0,04 Å.

2. Oxyde multimétallique selon la revendication 1, dans lequel b vaut 0.

3. Oxyde multimétallique selon la revendication 1 ou 2, qui présente une surface spécifique selon BET de 3 à 250 m²/g_{.}

4. Oxyde multimétallique selon l'une quelconque des revendications 1 à 3, où M représente Ce ou Mn.

5. Utilisation d'un oxyde multimétallique selon l'une quelconque des reveridications 1 à 4 pour la préparation de précatalyseurs et de catalyseur pour l'oxydation partielle en phase gazeuse d'hydrocarbures aromatiques.

6. Précatalyseur qui pleut être transformé en un catalyseur pour l'oxydation partielle en phase gazeuse d'hydrocarbures aromatiques, constitué par un support inerte non poreux et au moins une couche qui y est appliquée, qui comprend un oxyde multimétallique salon l'une quelconque des revendications 1 à 4.

7. Précatalyseur selon la revendication 6, qui contient 5 à 25 % en poids, par rapport au poids total du précatalyseur, d'oxyde multimétallique.

8. Précatalyseur selon la revendication 6 ou 7, dont le matériau support inerte non poreux est constitué de stéatite.

9. Catalyseur pour l'oxydation partielle en phase gazeuse d'hydrocarbures aromatiques, constitué par un support inerte non poreux et au moins une couche qui y est appliquée, qui comprend comme masse catalytiquement active un bronze d'argent-oxyde de Vanadium, qui contient au moins un métal M choisi parmi le Ce et/ou le Mn, prouvant être préparé à partir d'une masse d'oxyde multimétallique selon la revendication 1 ou d'un précatalyseur selon la revendication 6 par traitement thermique à des températures supérieures jusqu'à 200°C à 650°C.

10. Catalyseur selon la revendication 9, le bronze d'argent-vanadium contenant du Ce ou du Mn.

11. Catalyseur selon la revendication 9 ou 10 présentant une couche, dont la masse catalytiquement active présente une surface BET de 2 à 100 m²/g.

12. Procédé pour la préparation d'aldéhydes, d'acides carboxyliques et/ou d'anhydrides d'acide carboxylique, dans lequel on met en contact un flux gazeux qui comprend un hydrocarbure aromatique et un gaz contenant de l'oxygène moléculaire, à une température augmentée, avec un catalyseur selon l'une quelconque des revendications 9 à 11.

13. Procédé selon la revendication 12, dans lequel on génère le catalyseur in situ à partir d'un précatalyseur selon l'une quelconque des revendication 6 à 8.

14. Procédé selon la revendication 12 ou 13, dans lequel on met en contact le mélange réactionnel obtenu ou une fraction de celui-ci avec au moins un autre catalyseur, dont la masse catalytiquement active contient du pentoxyde de Vanadium et de l'anatase.

15. Procédé selon la revendication 14, dans lequel on guide le flux gazeux consécutivement sur un lit d'un catalyseur situé en amont et un lit d'un catalyseur situé en aval, le lit du catalyseur situé en amont contenant un catalyseur selon la revendication 9 et le lit du catalyseur situé en aval contenant au moins un catalyseur dont la masse catalytiquement active contient du pentoxyde de Vanadium et de l'anatase.

16. Procédé selon la revendication 15, dans lequel la masse catalytiquement active du catalyseur situé en aval contient 1 à 40% en poids d'oxyde de Vanadium, calculé sous forme de V₂O₅, 60 à 99% en poids de dioxyde de titane, calculé sous forme de TiO2, jusqu'à 1% en poids d'un composé de césium, calculé sous forme de Cs, jusqu'à 1% en poids d'un composé de phosphore, calculé sous forme de P, et jusqu'à 10% en poids d'oxyde d'antimoine, calculé sous forme de Sb₂O₃.

17. Procédé selon la revendication 16, dans lequel le lit du catalyseur situé en aval comprend au moins deux couches de catalyseurs, dont la masse catalytiquement active présente des teneurs en Cs différentes, la teneur en Cs diminuant dans le sens de l'écoulement du flux gazeux.

18. Procédé selon l'une quelconque des revendication 12 à 17, dans lequel on oxyde comme hydrocarbure aromatique l'o-xylène ou le naphtalène ou des mélanges d'oxylène et de naphtalène en anhydrite de l'acide phtalique.
